# EUROPEAN PATENT APPLICATION

(11) **EP 2 204 358 A1**
(43) Date of publication of application: **07.07.2010**
(21) Application number: 08022118.7
(22) Date of filing: 19.12.2008
(51) Int. Cl.: C07C 67/03, C10M 105/38, C07H 13/06

(54) **Process for making polyol esters with improved colour and odour quality**

(71) Applicant: Cognis IP Management GmbH, 40589 Düsseldorf (DE)
(72) Inventor: Mainx, Hans-Georg, 42799 Leichlingen (DE); Hofer, Peter, 40589 Düsseldorf (DE); Busch, Stefan, 40597 Düsseldorf (DE); Mahnke, Eike Ulf, 42553 Velbert (DE)
(74) Representative: Fabry, Bernd

(57) **Abstract**

Suggested is an improved process for making polyol esters with improved colour quality by transesterification of polyols or their alkoxylation products with fatty acid alkyl esters, which is **characterised in that** the reaction is carried out in the presence of a reducing mineral or organic acid as a catalyst selected from the group consisting of
(i) sulphuric or sulphonic acids with an oxidation value of sulphur of less than 6 or their salts, and/or
(ii) phosphoric or phosphonic acids with an oxidation value of phosphor of less than 5 or their salts.

## Description

### Field of the invention

The present invention is related to the area of polyol esters and refers to a new process for obtaining products with improved colour and odour quality using specific transesterification catalysts, new esters of alkoxylated carbohydrates and their use in various areas of application.

### Background of the invention

Polyol esters, like for example fatty acid esters of pentaerythrol, are well known products of organic chemistry which are employed in various areas of application, as for example lubricants or cosmetic thickeners. It is common to obtain these products from transesterification of polyols and fatty acid esters, in particular fatty acid methyl esters or glycerides. Typically, alkaline catalysts, in particular alkaline or alkaline earth oxides, hydroxides or methylates [US 4,942,228 (P&G)] as well as metal organic compounds like e.g. dibutyl tin diacetate are employed. In order to achieve a sufficient degree of transesterification it is necessary to conduct the reaction at high temperatures up to 250 °C. As a result, the products thus obtained show dark colours and exhibit undesired odours, what makes an after-treatment by means of bleaches and reducing agents mandatory. Unfortunately, often even intensive bleaching operation will not provide products of acceptable colour. This is particularly the case for the production of heat-sensitive carbohydrate esters.

It has therefore been the object of the present invention to develop a new process for producing polyol esters in particular for making products based on heat-sensitive starting materials, which leads to products of improved colour and odour quality. Another object of the invention has been to use such process for making carbohydrate esters which are not obtainable by the processes known from the state of the art.

### Detailed description of the invention

The present invention refers to an improved process for making polyol esters with improved colour quality by transesterification of polyols or their alkoxylation products with fatty acid esters, which is **characterised in that** the reaction is carried out in the presence of a reducing mineral or organic acid as a catalyst selected from the group consisting of
(i) sulphuric or sulphonic acids with an oxidation value of sulphur of less than 6 or their salts, and/or
(ii) phosphoric or phosphonic acids with an oxidation value of phosphor of less than 5 or their salts.

Surprisingly it has been observed that using the catalysts according to the invention reaction can be conducted under milder conditions, in particular lower temperatures and neutral pH value, allowing to produce light coloured esters without unwanted odours also by using heat-sensitive starting materials like for example carbohydrates and their alkoxylation products. Another advantage of the present invention is that the esters are free of traces of heavy metal catalysts like for example tin. This allows using the products also in areas like cosmetics or nutrition where the presence of heavy metals even in traces is inadmissible.

### Polyols

Suitable polyols preferably contain 2 to 15 carbon atoms and at least two hydroxyl groups. The polyols may contain other functional groups, more especially amino groups, or may be modified with nitrogen. Typical examples are
○ glycerol;
○ alkylene glycols such as, for example, ethylene glycol, diethylene glycol, propylene glycol, butylene glycol, hexylene glycol and polyethylene glycols with an average molecular weight of 100 to 1000 Dalton;
○ technical oligoglycerol mixtures with a degree of self-condensation of 1.5 to 10, such as for example technical diglycerol mixtures with a diglycerol content of 40 to 50 % by weight;
○ methylol compounds such as, in particular, trimethylol ethane, trimethylol propane, trimethylol butane, pentaerythritol, dipentaerythritol, neopentylglycol;
○ lower alkyl glucosides, particularly those containing 1 to 8 carbon atoms in the alkyl group, for example methyl and butyl glucoside;
○ sugar alcohols containing 5 to 12 carbon atoms, for example mannitol, sorbitol or its dehydrogenation product ("sorbitan");
○ carbohydrates containing 5 to 12 carbon atoms, for example glucose, sucrose, fructose, sorbit, dextrose, maltose, maltotriose or saccharose;
○ amino sugars, for example glucamine;
○ dialcohol amines, such as diethanol amine or 2-aminopropane-1,3-diol.

Also suitable starting materials are adducts of on average about to about 50, preferably about 5 to about 40 and more preferably about 10 to about 30 moles ethylene oxide and/or propylene oxide to said polyols. The alkoxylation products may show a blockwise or statistical distribution of the alkylene oxide units.

### Fatty acid esters

Typically said fatty acid esters forming the other component of the polyol esters, follow general formula (I),

R¹CO-OR² (I)

in which R¹CO represents a linear or branched, saturated or unsaturated, optionally hydroxy-functionalised acyl radical having about 6 to about 30 carbon atoms and 0 or 1 to 3 double bonds, and R² represents an alkyl radical having 1 to about 6 carbon atoms or the residue of glycerol. The fatty acid esters suitable to be used in the inventive process therefore represent either alkyl esters or (mono/di/tri) glycerides.

Typical examples of suitable esters can be chosen from methyl, ethyl, propyl, butyl, pentyl or hexyl esters of capronic acid, caprylic acid, caprinic acid, lauric acid, myrystic acid, palmitic acid, palmoleic acid, stearic acid, isostearic acid, oleic acid, elaidinic acid, linoleic acic, conjugated linoleic acid, linolenic acid, 12-hydroxy stearic acid, ricinoleic acid, gadoleic acid, arachidonic acid, behenic acid, Erucic acid and their technical mixtures like for example tallow acid, coco fatty acid, palm fatty acid, sunflower acid, or soy acid. Instead of the alkyl esters mono-, di- and/or triglycerides of the same fatty acids can also be used for transesterification. Finally, it is also possible to use natural fats and oils, for example sunflower or soy oil for the same purpose.

### Catalysts

The nature of the catalysts for the transesterification is critical and forms the centre of the invention. Typical examples for reducing mineral or organic are sulphuric or sulphonic acids selected from the group consisting of sulphurous acid, dithionic acid, sulphinic acid and organic sulphinic acids and their alkali or alkaline earth salts. It is also possible to apply mineral or organic acids represent phosphorous or phosphonic acids selected from the group consisting of phosphorous acid, diphosphorous acid, hypophosphorous acid, and hypodiphosphorous acid or their alkaline or alkaline earth salts. Preferred salts are potassium salts.

### Transesterification

Transesterification is conducted in a manner known from organic chemistry. Typically one component - usually the one being less expensive or easier to separate - is used in molar excess. Typically, the catalysts are prepared in-situ: stoichiometrical amounts of mineral acid and alkali hydroxide are added to the ester/polyol-mixture. It has been found advantageous to use the catalysts in concentrations of about 0.05 to about 1 % b.w., preferably about 0.1 to about 0.5 % b.w. calculated on the transesterification starting materials. Once the catalyst has been added, the mixture is heated up to the final temperature either under nitrogen or vacuum, depending on the reactants. Suitable reaction temperatures are in the range of about 120 to about 200 °C, preferably about 150 to about 180 °C. It is preferred to conduct the transesterification under reduced pressure, for example 1 to about 300 mbar and preferably about 10 to about 100 mbar. Different to the catalysts known from the state of the art the process according to the present invention can be conducted under mild conditions, preferably at pH values in between about 6 and about 8.. In particular running the reaction under neutral pH conditions was surprising and has not been expected. The esters obtained by this procedure are light coloured and do not need any bleaching after the reaction.

### Carbohydrate esters

Another object of the present invention covers certain carbohydrate esters which have not been prepared before due to the fact that the starting materials have been found too heat sensitive for surviving conventional transesterification processes. In particular the present invention also claims
adducts of on average 1 to about 80, preferably about 5 to about 60 and more preferably about 10 to about 50 moles ethylene oxide and/or propylene oxide to carbohydrates selected from the group consisting of
○ dextrose,
○ saccharose,
○ maltose and
○ maltotriose.

A particular preferred product is an adduct of on average 40 moles ethylene oxide and 6 moles propylene oxide to saccharose tetraoleate.

### Industrial application

Polyol esters obtainable by the process of the present invention in general and the new carbohydrate esters cited above in general are useful for quite a number of different applications. The present invention therefore also covers their use
○ for making agrochemical formulations;
○ for making blended lubes;
○ for making coated additives;
○ for making paint additives;
○ for making cosmetic and/or pharmaceutical compositions; and
○ as food additives.

### Examples

### Example 1

### Transesterification in the presence of hypophosphites

475 g (440 g active, 0,36 mol) of an adduct of on average 20 moles propylene oxide to saccharose were mixed with 276 g (1,1 mol) of a technical grade C₁₂-C₁₈ fatty acid methyl ester (Edenor^{®} MeC12-18, Cognis GmbH) and 15 g of a 25 % solution of potassium hypophosphite. The mixture was set under a 300 mbar vacuum and heated up slowly under stirring to 180°C. After the removal of the water the reaction started vigorously at a temperature of about 140 to 150 °C. After the removal of the first large amount of methanol the vacuum was slowly reduced to < 1 mbar and the reaction mixture was kept under these conditions for another 3 to 4 hours. Once the reaction was completed the final ester was cooled to room temperature.
- Yield:: approx. 670 g ester
- Appearance:: clear yellowish liquid with a fatty smell
- pH-value:: 6.2

The following figure represents the MALDI-MS product of the obtained ester:

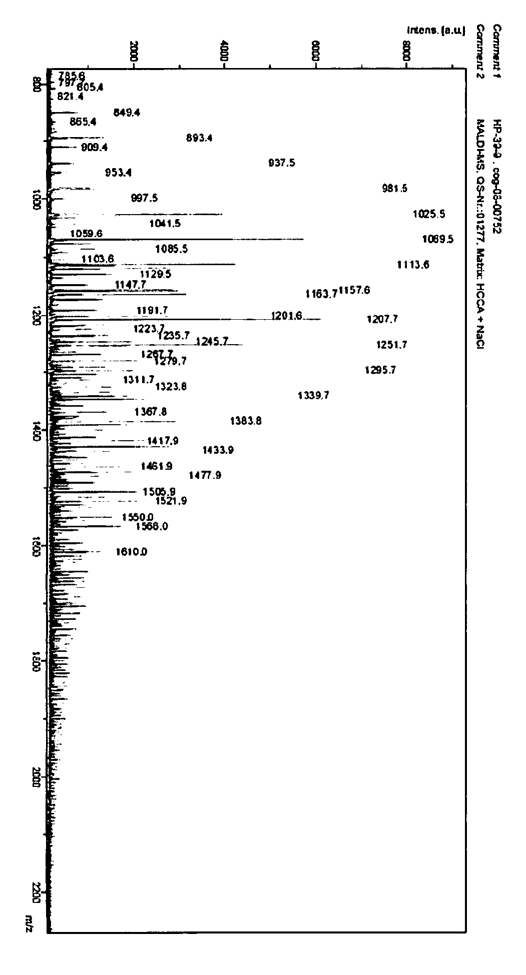

### Comparison Examples C1 and C2

### Transesterification in the presence of other catalysts

Inventive example 1 was repeated replacing the hypophosphite catalyst by (a) 0.3 % b.w. potassium hydroxide and (b) 0.3 % b.w. sodium methylate. In both experiments dark brown to black liquids with a smoky smell were obtained

### Example 2

### Transesterification in the presence of hypophosphites

Inventive example 1 was repeated using 85 g trimethylol propane, 565 g of a sunflower fatty acid methyl ester and 15 g of a 25 % b.w. solution of the potassium salt of hypophosphorous acid as the catalyst. One obtained a yield of 620 g ester in the form of clear colourless liquid with a fatty smell.

## Claims

1. Process for making polyol esters with improved colour quality by transesterification of polyols or their alkoxylation products with fatty acid esters, **characterised in that** the reaction is carried out in the presence of a reducing mineral or organic acid as a catalyst selected from the group consisting of
(i) sulphuric or sulphonic acids with an oxidation value of sulphur of less than 6 or their salts, and/or
(ii) phosphoric or phosphonic acids with an oxidation value of phosphor of less than 5 or their salts.

2. Process according to Claim 1, **characterised in that** said polyols are selected from the group consisting of glycerol, alkylene glycols, technical oligoglycerol mixtures with a degree of self-condensation of 1.5 to 10, methylol compounds, lower alkyl glucosides, sugar alcohols containing 5 to 12 carbon atoms, carbohydrates containing 5 to 12 carbon atoms, amino sugars and dialcohol amines.

3. Process according to Claims 1 and/or 2, **characterised in that** said polyols represent adducts of on average 1 to 80 moles ethylene oxide and/or propylene oxide to glycerol, alkylene glycols, technical oligoglycerol mixtures with a degree of self-condensation of 1.5 to 10, methylol compounds, lower alkyl glucosides, sugar alcohols containing 5 to 12 carbon atoms, carbohydrates containing 5 to 12 carbon atoms, amino sugars and dialcohol amines.

4. Process according to any of the preceding Claims 1 to 3, **characterised in that** said fatty acid esters follow general formula (I),
R¹CO-OR² (I)
in which R¹CO represents a linear or branched, saturated or unsaturated, optionally hydroxy-functionalised acyl radical having 6 to 30 carbon atoms and 0 or 1 to 3 double bonds, and R² represents an alkyl radical having 1 to 6 carbon atoms or the residue of glycerol.

5. Process according to any of the preceding Claims 1 to 4, **characterised in that** said reducing mineral or organic acids represent sulphuric or sulphonic acids selected from the group consisting of sulphurous acid, dithionic acid, sulphinic acid and organic sulphinic acids and their alkali or alkaline earth salts.

6. Process according to any of the preceding Claims 1 to 5, **characterised in that** said reducing mineral or organic acids represent phosphorous or phosphonic acids selected from the group consisting of phosphorous acid, diphosphorous acid, hypophosphorous acid, and hypodiphosphorous acid or their alkaline or alkaline earth salts.

7. Process according to any of the preceding Claims 1 to 6, **characterised in that** said reducing mineral or organic acids are used in amounts of 0.05 to 1 % b.w. calculated on the transesterification starting materials.

8. Process according to any of the preceding Claims 1 to 7, **characterised in that** the transesterification is conducted at a pH value of 6 to 8.

9. Adducts of on average 1 to 80 moles ethylene oxide and/or propylene oxide to carbohydrates selected from the group consisting of dextrose, saccharose, maltose and maltotriose.

10. Use of polyol esters according to Claim 9 for making agrochemical formulations.

11. Use of polyol esters according to Claim 9 for making blended lubes.

12. Use of polyol esters according to Claim 9 for making coated additives.

13. Use of polyol esters according to Claim 9 for making paint additives.

14. Use of polyol esters according to Claim 9 for making cosmetic and/or pharmaceutical compositions.

15. Use of polyol esters according to Claim 9 as food additives.
